(19) Europäisches Patentamt
European Patent Office
Office européen des brevets

(11) EP 1 925 941 B1

(12) **EUROPEAN PATENT SPECIFICATION**

(45) Date of publication and mention of the grant of the patent:
**28.11.2012 Bulletin 2012/48**

(21) Application number: **06782407.8**

(22) Date of filing: **01.08.2006**

(51) Int Cl.:
***G01N 33/574*** (2006.01) ***A61K 31/404*** (2006.01)
***A61K 31/47*** (2006.01) ***A61K 39/395*** (2006.01)
***A61K 45/00*** (2006.01) ***A61P 35/00*** (2006.01)
***C12Q 1/68*** (2006.01) ***G01N 33/15*** (2006.01)
***G01N 33/50*** (2006.01) ***C12N 15/09*** (2006.01)

(86) International application number:
**PCT/JP2006/315563**

(87) International publication number:
**WO 2007/015569 (08.02.2007 Gazette 2007/06)**

(54) **METHOD FOR PREDICTION OF THE EFFICACY OF VASCULARIZATION INHIBITOR**

VERFAHREN ZUR VORHERSAGE DER WIRKSAMKEIT EINES VASKULARISIERUNGSINHIBITORS

PROCÉDÉ DE PRÉDICTION DE L'EFFICACITÉ D'UN INHIBITEUR DE VASCULARISATION

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **01.08.2005 JP 2005223440**

(43) Date of publication of application:
**28.05.2008 Bulletin 2008/22**

(73) Proprietor: **Eisai R&D Management Co., Ltd.**
**Tokyo 112-8088 (JP)**

(72) Inventors:
- **MATSUI, Junji,**
**c/o EISAI CO., LTD.**
**Tsukuba-shi,**
**Ibaraki 300-2635 (JP)**
- **SEMBA, Taro,**
**c/o EISAI CO., LTD.**
**Tsukuba-shi,**
**Ibaraki 3000-2635 (JP)**

(74) Representative: **Woods, Geoffrey Corlett**
**J A Kemp**
**14 South Square**
**Gray's Inn**
**London WC1R 5JJ (GB)**

(56) References cited:
- **BENJAMIN L E ET AL: "Selective ablation of immature blood vessels in established human tumors follows vascular endothelial growth factor withdrawal." THE JOURNAL OF CLINICAL INVESTIGATION JAN 1999 LNKD- PUBMED: 9916127, vol. 103, no. 2, January 1999 (1999-01), pages 159-165, XP009133357 ISSN: 0021-9738**
- **BERGERS GABRIELE ET AL: "Benefits of targeting both pericytes and endothelial cells in the tumor vasculature with kinase inhibitors" JOURNAL OF CLINICAL INVESTIGATION, AMERICAN SOCIETY FOR CLINICAL INVESTIGATION, US LNKD- DOI: 10.1172/JCI200317929, vol. 111, no. 9, 1 May 2003 (2003-05-01), pages 1287-1295, XP002317731 ISSN: 0021-9738**
- **ERBER RALF ET AL: "Combined inhibition of VEGF and PDGF signaling enforces tumor vessel regression by interfering with pericyte-mediated endothelial cell survival mechanisms" FASEB JOURNAL, FED. OF AMERICAN SOC. FOR EXPERIMENTAL BIOLOGY, US, vol. 18, no. 2, 4 December 2004 (2004-12-04), pages 338-340, XP002548466 ISSN: 0892-6638 [retrieved on 2009-10-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- **MCCARTY MARYA F ET AL: "ZD6474, a vascular endothelial growth factor receptor tyrosine kinase inhibitor with additional activity against epidermal growth factor receptor tyrosine kinase, inhibits orthotopic growth and angiogenesis of gastric cancer." MOLECULAR CANCER THERAPEUTICS SEP 2004 LNKD- PUBMED: 15367698, vol. 3, no. 9, September 2004 (2004-09), pages 1041-1048, XP009133243 ISSN: 1535-7163**
- **KURZ H. ET AL.: 'Pericytes in experimental MDA-MB231 tumor angiogenesis' HISTOCHEMISTRY AND CELL BIOLOGY vol. 117, no. 6, June 2002, pages 527 - 534, XP003007852**
- **MORIKAWA S. ET AL.: 'Kekkan Shinsei to Shuhi Saibo' THE CELL vol. 37, no. 4, 2005, pages 164 - 168, XP003007853**

## Description

### TECHNICAL FIELD

The present invention relates to a novel method for predicting the effect of angiogenesis inhibitors (Vascularization Inhibitors), such as substances having vascular endothelial growth factor (hereinafter, sometimes referred to as "VEGF") inhibitory activity (hereinafter, sometimes referred to as "VEGF inhibitors").

### BACKGROUND ART

Clinical trials have made it clear that angiogenesis inhibitors are useful as antitumor agents. For example, bevacizumab that is a neutralizing antibody against VEGF playing an important role among angiogenic processes is reported to have shown an antitumor effect against colorectal cancer in clinical trials[(1)].

4-(3-chloro-4-(cyclopropylaminocarbonyl)-aminophenoxy)-7-methoxy-6-quinolinecarboxamide is reported as an angiogenesis inhibitor (2 and 3)

Evaluating the effect of angiogenesis inhibitors , determining the effective dose of angiogenesis inhibitors and predicting the effect of angiogenesis inhibitors prior to administration thereof are very useful for efficiently performing treatment with angiogenesis inhibitors and for contributing to the improvement of patients' QOL[(4)]. With respect to the former two matters, a great number of researches are now being carried out[(5)]. Specifically, methods such as dynamic contrast-enhanced magnetic resonance imaging (DCE-MRI), positron emission tomograpy (PET), interstitial fluid pressure and serum VEGF are reported. Among all, DCE-MRI is believed to be effective as a method for evaluating the effect of angiogenesis inhibitors[(6)].

On the other hand, predicting the effect of angiogenesis inhibitors prior to administration thereof is very beneficial and important to patients for avoiding the administration of inefficient medicine and reducing adverse effect[(4)]. However, no effective method for predicting the effect of angiogenesis inhibitors prior to administration thereof has been found yet.

Benajmin et al. (1999) J. Clin. Invest. 103, 159-165 discloses that selective ablation of immature blood vessels in established human tumours follows vascular endolethial growth factor withdrawal. Features that distinguish tumour vasculatures from normal blood vessels were sought to enable the destruction of preformed tumour vessels. It was shown that blood vessels in both a xenografted tumor and primary human tumors contain a sizable fraction of immature blood vessels that have not yet recruited pericytes. These immature vessels were selectively obliterated as a consequence of vascular endolethial growth factor (VEGF) withdrawal.

### REFERENCES

(1) Bevacizumab plus irinotecan, fluorouracil, and leucovorin for metastatic colorectal cancer, N Engl J Med. 2004, 350, 2335-42

(2) WO 02/32872

(3) WO 2004/080462

(4) Inhibition of vascular endothelial growth factor (VEGF) signaling in cancer causes loss of endothelial fenestrations, regression of tumor vessels, and appearance of basement membrane ghosts. Am J Pathol., 2004, 165, 35-52

(5) Direct evidence that the VEGF-specific antibody bevacizumab has antivascular effects in human rectal cancer, Nature Medicine, 2004, 10, 145-147

(6) Dynamic contrast-enhanced magnetic resonance imaging as a biomarker for the pharmacological response of PTK787/ZK 222584, an inhibitor of the vascular endothelial growth factor receptor tyrosine kinases, in patients with advanced colorectal cancer and liver metastases: results from two phase I studies., J Clin Oncol., 2003, 21, 3955-64.


### DISCLOSURE OF THE INVENTION

Under such circumstances, the present invention has been made. It is an object of the invention to find a method for predicting the effect of angiogenesis inhibitors.

As a result of extensive and intensive researches toward the solution of the above problem, the present inventors have found out for the first time that the antitumor effect of angiogenesis inhibitors correlates with the number of those blood vessels which are coated with pericytes in the relevant tumor. The present inventors have also found out that it is possible to predict the antitumor effect of angiogenesis inhibitors by determining the number of those blood vessels which are covered with pericytes in the relevant tumor and using the resultant number as an indicator.

The present invention provides a method of predicting the antitumor effect of an angiogenesis inhibitor, comprising the following steps:

a first step of determining the number of blood vessels in a tumor sample that has been removed from a cancer patient and the number of those blood vessels which are covered with pericytes in the tumor sample; and
a second step of judging that a cancer patient is highly sensitive to the angiogenesis inhibitor when the ratio of the number of those blood vessels covered with pericytes in the tumor to the total number of blood vessels in the tumor is 25% or less,
wherein the angiogenesis inhibitor is one compound selected from the group consisting of:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide; and
5-(5-fluoro-2-oxo-1,2-dihydroindole-3-ylidenemethyl)-2,4-dimethyl-1H-pyrrole -3-carboxylic acid (2-diethylaminoethyl)amide,

or a pharmacologically acceptable salt of said compound, or a solvate of said compound or said salt.

In the method of the invention, the tumor is a tumor collected from the cancer patient.

In the method of the invention, the determination of the number of those blood vessels which are covered with pericytes may be performed by using as an indicator the expression of at least one substance selected from the group consisting of α-SMA, desmin, chondroitin sulfate proteoglycan 4, calponin, caldesmon and PDGF receptor. Among all, the expression of α-SMA and/or desmin is used preferably as an indicator. The determination of the number of those blood vessels which are covered with pericytes may be performed by, for example, an immunochemical method, *in situ* hybridization or quantitative RT-PCR.

Further, in the method of the invention, the determination of the number of blood vessels in the tumor may be performed by using as an indicator the expression of at least one substance selected from the group consisting of CD31, wVF, CD34, CD105, CXCR4, CD146, CD133, KDR and KIT. Among all, the expression of CD31 is used preferably as an indicator. The determination of the number of blood vessels in the tumor may be performed by, for example, an immunochemical method, *in situ* hybridization or quantitative RT-PCR.

In the method of the invention, the angiogenesis inhibitor is a VEGF receptor kinase inhibitor, preferably 4-(3-chloro-4-(cyclopropylaminocarbonyl)aminophenoxy)-7-methoxy-6-quinolinecarboxamide, a pharmacologically acceptable salt thereof, or a solvate of the compound or the salt.

According to the present invention, a method of predicting the antitumor effect of an angiogenesis inhibitor is provided.

More specifically, it has become possible to predict the antitumor effect of an angiogenesis inhibitor by determining the number of those blood vessels which are covered with pericytes in a tumor and using the resultant number as an indicator.

Since the method according to the present invention enables to predict the antitumor effect of an angiogenesis inhibitor without administering the agent to patients, it has become possible to select and treat those patients who are expected to show higher antitumor effect. Thus, contribution to patients' QOL has become possible.

## BRIEF DESCRIPTION OF THE DRAWINGS

Fig. 1 shows the correlation between the antitumor effect of 4-(3-chloro-4-(cyclopropylaminocarbonyl)aminophenoxy)-7-methoxy-6-quinolinecarboxamide and the number of those blood vessels which are covered with pericytes in tumor tissue in human cancer cell strain transplanted mouse models.
Fig. 2 shows the correlation between the antitumor effect of angiogenesis inhibitors and the number of those blood vessels which are covered with pericytes in human cancer cell lines transplanted mouse models. In Fig. 2, Compound 1 represents 4-(3-chloro-4-(cyclopropylaminocarbonyl)aminophenoxy)-7-methoxy-6-quinolinecarboxamide and Compound 2 represents 5-(5-fluoro-2-oxo-1,2-dihydroindole-3-ylidenemethyl)-2,4-dimethyl-1H-pyrrole-3-carboxylic acid (2-diethylaminoethyl)amide.
Fig. 3 shows the correlation between the antitumor effect of 4-(3-chloro-4-(cyclopropylaminocarbonyl)aminophenoxy)-7-medioxy-6-quinolinecarboxamide and a pericyte marker desmin in tumor tissue in human cancer cell lines subcutaneously transplanted mouse models.

BEST MODE FOR CARRYING OUT THE INVENTION

Hereinbelow, the embodiments of the present invention will be described. The following embodiments are provided only to illustrate the present invention, and they are not intended to limit the present invention only to these embodiments.

The present invention provides a method of prediction the antitumor effect of an angiogenesis inhibitor, comprising a step of determining the number of those blood vessels which are covered with pericytes in a tumor; and a step of judging whether or not a cancer patient is highly sensitive to the angiogenesis inhibitor by using as an indicator the resultant number of those blood vessels which are covered with pericytes.

1. Step of Determining the Number of Those Blood Vessels Which Are Covered with Pericytes in Tumor

In this step, the tumor is a tumor removed from the cancer patient. Such a tumor may be obtained by removing a tumor tissue from the cancer patient by surgical treatment (e.g., biopsy).

The size of tumor sample to be removed from cancer patients is not particularly limited. Any size may be used as long as the tumor sample allows determination of the number of those blood vessels covered with pericytes therein. For example, if the tumor is a solid cancer, the size of tumor sample to be removed may be a size of a tumor sample taken by biopsy (e.g., 2-3 mm) or a size of a tissue section removed with a surgical knife (e.g., the size of grain of rice).

The type of tumor used in the present invention is not particularly limited. For example, brain tumor, head&neck cancer, esophageal cancer, tongue cancer, lung cancer, breast cancer, pancreatic cancer, gastric cancer, cancer of the small intestine or duodenum, large bowel cancer (colon cancer, rectal cancer), bladder cancer, renal cancer, liver cancer, prostate cancer, uterine cancer, ovary cancer, thyroid cancer, gallbladder cancer, pharyngeal cancer, sarcoma (e.g., osteosarcoma, chondrosarcoma, Kaposi sarcoma, myosarcoma, angiosarcoma, fibrosarcoma or the like), leukemia (e.g., chronic myelogenous leukemia (CML), acute myelogenous leukemia (AML), chronic lymphocytic leukemia (CLL), acute lymphocytic leukemia (ALL), lymphoma, malignant lymphoma, multiple myeloma (MM) or the like), melanoma and so forth may be enumerated.

Pericytes exists surrounding blood capillaries and veins, and they are referred to perithelial cells.

Pericytes express $\alpha$-smooth muscle actin (hereinafter, sometimes referred to as "$\alpha$-SMA"), desmin, chondroitin sulfate proteoglycan 4 (hereinafter, sometimes referred to as "NG-2"), calponin, caldesmon (Characterization of smooth muscle cell and pericyte differentiation in the rat retina in vivo, Investigative. Ophthalmology. Visual Science. 45, 2795-2806, 2004), platelet-derived growth factor receptor (hereinafter, sometimes referred to as "PDGF receptor") (Cellular abnormalities of blood vessels as targets in cancer, Current Opinion in Genetics and Development, 15, 102-111. 2005), etc. and do not express factor VIII (A new method for isolation of smooth muscle cells from human umbilical cord arteries, Scand J. Clin. Lab. Invest. 57, 21-29, 1997) and GFAP (Localization of Brain Endothelial Luminal and Ablwninal Transporters with Immunogold Electron Microscopy, NeuroRx., 2, 27-43, 2005). Therefore, pericytes may be distinguished from other cells by examining the presence or absence of the expression of these substances.

The term "blood vessels (which are) covered with pericytes" means blood vessels which are totally or partially surrounded by pericytes.

The "number of blood vessels (which are) covered with pericytes" may be calculated, for example, as the number of blood vessels covered with pericytes per unit area in a tumor, or as the number of blood vessels covered with pericytes per unit volume in a tumor, or as the number of blood vessels covered with pericytes per unit weight.

In this step, the number of blood vessels covered with pericytes may be determined, for example, by using as an indicator the expression of a protein and/or mRNA which is expressed in pericytes.

Examples of proteins and/or genes expressed in pericytes include $\alpha$-SMA, desmin, chondroitin sulfate proteoglycan 4, calponin, caldesmon and PDGF receptor. Preferably, $\alpha$-SMA or desmin is used. For example, by measuring the expression of these proteins and/or mRNAs in tumor samples collected from patients, it is possible to obtain information (such as the types of proteins and/or genes expressed in the tumor samples, the presence or absence of expression thereof, or the expression levels thereof). Using this information as an indicator, it is possible to calculate the number of those blood vessels covered with pericytes.

Measurement of protein may be performed by such methods as immunochemical methods (e.g., immunohistochemical methods or Western blotting) or mass spectrometry. Preferably, immunochemical methods are used. Particularly preferably, immunohistochemical methods are used. These methods may be performed according to conventional procedures.

On the other hand, measurement of mRNA may be performed by such methods as *in situ* hybridization, Northern blotting, DNA microarray, RT-PCR and quantitative RT-PCR. Preferably, *in situ* hybridization and quantitative RT-PCR may be enumerated. These methods may be performed according to conventional procedures.

*In situ* hybridization may be performed, for example, according to the method described in "Jikkenn Igaku Bessatu, Shin-Idenishikogaku Handbook" (Experimental Medicine Special Issue, New Genetic Engineering Handbook),

Chapter 4, published by Yodosha in 2003.

Hereinbelow, one example of a method of determining the number of blood vessels covered with pericytes will be described.

The number of blood vessels covered with pericytes may be determined by an immunohistochemical method using as an indicator the expression of a protein(s) expressed specifically in pericytes.

The immunohistochemical method may be performed according to conventional procedures ("Saibo-Kogaku Bessatu, Me de Mirujikkenn note series, Bio-Jikkenn Illustrated Vol. 5, Tanpaku-nante-Kowakunai" (Special Issue of Cell Engineering, Visual Experimental Note Series, Illustrated Biological Experiments, Vol. 5 "Who's Afraid of Proteins"), Chapter 5, Immunostaining, pp. 127-163, published by Shujunsha Co., Ltd., 1997).

First, tissue sections are prepared from tumor samples removed from cancer patients. Examples of tissue sections include frozen sections and paraffin sections.

Tumor samples removed from patients may be either untreated or treated for fixation. The tumor samples may be embedded with OCT compound or the like.

Fixation treatment may be performed with formaldehyde, preferably 4% PFA/PBS(-). Then, the formaldehyde may be replaced with 20% sucrose/phosphate buffer or the like.

Various conditions for these operations may be selected appropriately depending on the protein to be measured and the antibody to be used.

The tissue section may be retained on a slide glass and pretreated to make staining possible. The method of this pretreatment is not particularly limited and may be appropriately selected depending on the protein to be measured and the antibody to be used. For example, the tissue section may be pretreated with a solution containing xylene, formaldehyde, acetone, methanol, etc. Alternatively, the tissue section may be pretreated with a solution containing BSA, Triton-X100, tween 20, skim milk, casein, etc.

Subsequently, the pretreated tissue section is contacted with an antibody that recognizes a protein to be measured (hereinafter, sometimes referred to as the "primary antibody"). The primary antibody may be a commercially available antibody or may be prepared. The primary antibody may be labeled with a labeling agent or may not be labeled. When the primary antibody is not labeled, an antibody that recognizes the primary antibody (hereinafter, sometimes referred to as the "secondary antibody") may be contacted therewith. The secondary antibody is preferably labeled with a labeling agent. Examples of the labeling agent include enzymes (such as alkaline phosphatase, peroxidase, glucose oxidase, β-galactosidase), fluorescent substances (such as FITC (fluorescein isothiocyanate), Alexa488, PE, Rhodamin, Texas Red, Cy3, Cy5, allophycocyanin, PharRed, DsRed, AmCyan, ZsGreen, ZsYellow, AsRed, HcRed) and biotin. When the labeling agent is biotin, avidin or streptavidin may be further contacted. Such avidin or streptavidin is preferably labeled with a labeling agent. Examples of the labeling agent include enzymes (such as alkaline phosphatase, peroxidase, glucose oxidase, β-galactosidase) and fluorescent substances (such as FITC, Alexa488, PE, Rhodamin, Texas Red, Cy3, Cy5, allophycocyanin, PharRed, DsRed, AmCyan, ZsGreen, ZsYellow, AsRed, HcRed). Various conditions of reactions (such as reaction solution, antibody concentration, reaction time, reaction temperature, washing procedure, etc.) may be appropriately selected depending on the protein to be measured and the antibody to be used.

When the labeling agent is an enzyme, a substrate and/or a coloring reagent is contacted with the tissue section for coloring. By observing this coloring, it is possible to determine the number of blood vessels coated with pericytes.

When the enzyme is peroxidase, a substrate such as $H_2O_2$ and a coloring reagent such as diaminobenzidine (DAB) may be contacted with the tissue section.

When the enzyme is alkaline phosphatase, a substrate such as 5-bromo-4-chloro-3-indolyl phosphate and a coloring reagent such as nitrobluetetrazorium may be contacted with the tissue section. When the enzyme is alkaline phosphatase, it is also possible to perform a chemiluminescent reaction by contacting a coloring substrate such as CSPD (disodium 3-(4-methoxyspiro{1,2-dioxetane-3,2'-(5'-chloro)tricyclo[3.3.1.1$^{3,7}$]-decan}-4-yl)phenylphosphate) with the tissue section.

When the labeling agent is a fluorescent substance, the number of blood vessels coated with pericytes may be measured by irradiating the tissue section with excitation light for luminescence and observing the resultant fluorescence.

Further, the treated tissue section may be nuclear stained with hematoxylin or methyl green.

Further, the treated tissue section may be mounted with an aqueous mounting medium.

Thus, the number of blood vessels covered with pericytes per unit area in tumor may be calculated. Alternatively, the number of blood vessels covered with pericytes may be calculated as a value per unit volume of the tumor or as a value per unit weight of the tumor.

Hereinbelow, another example of the method of determining the number of blood vessels covered with pericytes will be described.

It is possible to determine the number of blood vessels covered with pericytes by quantitative RT-PCR using as an indicator the expression of an mRNA expressed specifically in pericytes.

First, RNA is purified from a tumor sample removed from a cancer patient.

TRIZOL reagent (Invitrogen) is added to the tumor sample to homogenize the tumor tissue. Subsequently, chloroform is added to the homogenized tumor. The resultant solution is shook and agitated vigorously for 15 sec, left at room temperature for 2 to 3 min, and then centrifuged (12,000x, 10 min, 4°C). After centrifugation, the aqueous layer is tranferrred to a fresh tube. To this tube, isopropyl alcohol is added. After leaving at room temperature for 10 min, the tube was centrifuged (12,000x, 10 min, 4°C). The resultant precipitate is washed with 75% ethanol to thereby purify RNA.
Quantitative RT-PCR may be performed as described below using gene-specific probes (TaqMan Gene Expression Assays Mixture (ASSAYS-ON-DEMAND); Applied Biosystems) and ABI Prism 7900 Sequence Detection System (Perkin-Elmer Applied Biosystems).
Operation may be performed in two-stages, i.e., reverse transcription reaction and PCR reaction. Reverse transcription reaction (the first stage) is performed by adding dNTP, oligo $d(T)_{16}$ primer, RNase Inhibitor and Multiscribe Reverse Transcriptase (Perkin-Elmer Applied Biosystems) to the resultant RNA, retaining the mixture at 25°C for 10 min and then heating at 48°C for 30 min. The reaction is terminated by heating the reaction solution at 95°C for 5 min.
The resultant cDNA is subjected to the PCR reaction at the second stage. The PCR reaction is performed in a reaction system comprising, for example, 4 ng of cDNA, 1 xSYBR PCR buffer, 3 mM $MgCl_2$, 200 $\mu$M each of dATP, dCTP and dGTP, 400 $\mu$M dUTP, 200 nM primer pair, 0.01 U/$\mu$l AmpErase UNG and 0.025 U/$\mu$l AmpliTaq Gold DNA Polymerase (Perkin-Elmer Applied Biosystems). The reaction conditions were as follows: 50°C for 2 min and 95 °C for 10 min, followed by 40 cycles of 95°C for 20 sec, 55°C for 20 sec and 72°C for 30 sec. Primers and probes may be designed using Primer Expression (Perkin-Elmer Applied Biosystems), for example. Alternatively, TaqMan Gene Expression Assays mixture (ASSAYS-ON-DEMAND; Applied Biosystems) may be used as primers and probes. Comparison of a plurality of samples may be performed by correcting the quantitatively determined values by the mRNA level of a housekeeping gene whose transcription level vary little among samples (preferably, GAPDH, $\beta$-action, 18S ribosomal RNA or the like).
Further, it is preferable to determine in advance the number of blood vessels in a tumor (i.e., the total number of the blood vessels covered with pericytes and the blood vessels not covered with pericytes) using as an indicator the expression of a protein(s) and/or mRNA(s) expressed specifically in vascular endothelial cells. Examples of proteins and/or genes (mRNAs) expressed specifically in vascular endothelial cells include CD31, wVF (von Willebrand Factor), CD34, CD105, CXCR4, CD146, CD133, KDR (VEGF receptor 2) and KIT (Vascular and haematopoietic stem cells: novel targets for anti-angiogenesis therapy? Nature Reviews Cancer, 2, 826-35, 2002). Preferably, CD31 is used. The number of blood vessels may be calculated by such methods as immunochemical methods, *in situ* hybridization or quantitative RT-PCR, in the same manner as in the determination of the number of blood vessels covered with pericytes.
At this point, it is possible to further improve the accuracy of judgment as to whether a cancer patient is highly sensitive to an angiogenesis inhibitor, by amending the number of those blood vessels covered with pericytes by the total number of blood vessels (i.e., the total number of the blood vessels covered with pericytes and the blood vessels not covered with pericytes). For example, a quotient obtained by dividing the number of those blood vessels covered with pericytes by the total number of blood vessels (i.e., the total number of the blood vessels covered with pericytes and the blood vessels not coated with pericytes) may be used as an indicator.

2. Step of Judging Whether or Not Cancer Patients Are Highly Sensitive to Angiogenesis Inhibitor

In this step, it is possible to judge whether or not cancer patients are highly sensitive to an angiogenesis inhibitor using as an indicator the number of those blood vessels covered with pericytes determined in the previous step. Then, from the result of judgment on the sensitivity, it is possible to predict the antitumor effect of the angiogenesis inhibitor.
In this step, as the number of those blood vessels covered with pericytes, the following values may be used as an indicator, for example: (i) the number of those blood vessels covered with pericytes per unit area in tumor; (ii) the number of those blood vessels covered with pericytes per unit volume in tumor; (iii) the number of those blood vessels covered with pericytes per unit weight in tumor; and (iv) the ratio of the number of those blood vessels covered with pericytes to the total number of blood vessels (i.e., the total number of the blood vessels covered with pericytes and the blood vessels not covered with pericytes) in tumor.
When the number of those blood vessels covered with pericytes in tumor is small, it is possible to judge that the relevant cancer patient is highly sensitive to the angiogenesis inhibitor. On the other hand, when the number of those blood vessels covered with pericytes in the tumor collected from the cancer patient is large, it is possible to judge that the relevant cancer patient is not highly sensitive to the angiogenesis inhibitor.
The expression "when the number of those blood vessels covered with pericytes in tumor is small" means, for example, that the ratio of the number of those blood vessels covered with pericytes to the total number of blood vessels (i.e., the total number of the blood vessels covered with pericytes and the blood vessels not covered with pericytes) is 25% or less, preferably 20% or less, more preferable 15% or less, particularly preferably 10% or less. The expression "when the number of those blood vessels covered with pericytes is large" means, for example, those cases which do not fall under the above-described cases of "when the number of those blood vessels covered with pericytes in tumor

is small".

In the present invention, the major purpose of prediction of antitumor effect is to know how much antitumor effect an angiogenesis inhibitor will produce in cancer patients prior to the administration thereof.

When a cancer patient has been judged highly sensitive to an angiogenesis inhibitor, it is possible to predict that the angiogenesis inhibitor will produce higher antitumor effect in the patient. Cases where higher antitumor effect will be produced include those cases, for example: antitumor effect higher than average antitumor effect in patients with similar symptoms can be expected; antitumor effect higher than the effect in other patients with the same cancer species can be expected; or antitumor effect higher than the effect in patients with other cancer species can be expected.

However, as described later, angiogenesis inhibitor inherently have an angiogenesis inhibitory effect. Therefore, even when a cancer patient has been judged not highly sensitive to an angiogenesis inhibitor, it should not be construed that the relevant angiogenesis inhibitor will produce no antitumor effect.

As another embodiment of the present invention, a method of selecting those patients who are highly sensitive to an angiogenesis inhibitor by using as an indicator the number of those blood vessels covered with pericytes is provided. As described above, when the number of those blood vessels covered with pericytes is small, the relevant patient can be judged highly sensitive to the angiogenesis inhibitor. Therefore, such patients may be selected as patients with high sensitivity to the angiogenesis inhibitor

As still another embodiment of the present invention, the invention provides a method of analyzing the sensitivity to an angiogenesis inhibitor by using as an indicator the number of those blood vessels covered with pericytes and classifying patients according to the results of this analysis. Briefly, in the method of the present invention, it is possible to analyze sensitivity to an angiogenesis inhibitor as described above and classify patients according to the results of this analysis. For example, patients may be classified into a group with a large number of blood vessels covered with pericytes and a group with a small number of blood vessels covered with pericytes.

As still another embodiment of the present invention, the invention provides a method of selecting patients to be administered with an angiogenesis inhibitor by using as an indicator the number of those blood vessels covered with pericytes. Patients who have a small number of blood vessels covered with pericytes are expected to show high sensitivity to the angiogenesis inhibitor. Therefore, these patients are selected as patients to be administered with the angiogenesis inhibitort.

As still another embodiment of the present invention, a method of predicting the therapeutic effect of an angiogenesis inhibitor in a patient by using as an indicator the number of those blood vessels covered with pericytes is provided. In the method of the present invention, when the number of those blood vessels covered with pericytes is small, it can be judged that the relevant patient will show high sensitivity to the angiogenesis inhibitor. Therefore, it is possible to predict that the therapeutic effect of the angiogenesis inhibitor will be high in the patient.

Further, the present invention include a method of evaluating the number of those blood vessels covered with pericytes in a patient, in order to predict the degree of sensitivity of the patient to an angiogenesis inhibitor. The method of evaluation is as described in sub-section 1. above.

In the present step, examples of angiogenesis inhibitors are as described later. Preferably, the angiogenesis inhibitor is 4-(3-chloro-4-(cyclopropylaminocarbonyl)-aminophenoxy)-7-methoxy-6-quinolinecarboxamide, a pharmacologically acceptable salt thereof, or a solvate of the compound or the salt.

The method according to the present invention may be used in order to predict the degree of efficacy of an angiogenesis inhibitor in a patient prior to the administration of the agent to the patient. Also, the method of the present invention makes it possible to select those patients in whom higher effect of the angiogenesis inhibitor can be expected and treat them. Thus, the present invention is clinically very useful.

3. Angiogenesis Inhibitors

In the present invention, the angiogenesis inhibitors are VEGF receptor kinase inhibitors, selected from the group consisting of

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide; and
5-(5-fluoro-2-oxo-1,2-dihydroindole-3-ylidenemethyl)-2,4-dimethyl-1H-pyrrole -3-carboxylic acid (2-diethylaminoethyl)amide,

or a pharmacologically acceptable salt of said compound, or a solvate of said compound or said salt.

The following five compounds:

4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinolinecarboxamide;

may be prepared by known methods. For example, those compounds may be prepared by the method described in WO 02/32872 or WO 2005/063713.

As a preferred such compound, 4-(3-chloro-4-(cyclopropylaminocarbonyl)aminophenoxy-7-methoxy-6-quinolinecarboxamide (see formula (I)) may be given. As one of the most preferable examples of VEGF receptor kinase inhibitors for use in the invention, the methanesulfonic acid salt of 4-(3-chloro-4-(cyclopropylaminocarbonyl)aminophenoxy-7-methoxy-6-quinolinecarboxamide may be given.

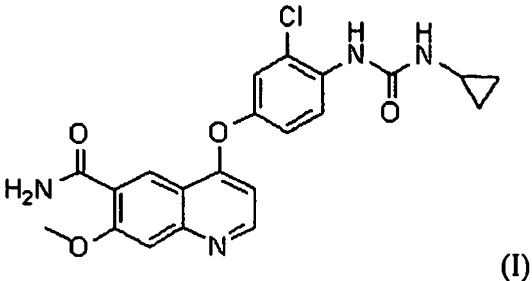

(I)

The other VEGF receptor kinase inhibitor compound is 5-(5-fluoro-2-oxo-1,2-dihydroindole-3-ylidenemethyl)-2,4-dimethyl-1H-pyrrole-3-carboxylic acid (2-diethylaminoethyl)amide (hereinafter, sometimes referred to as "SU11248". Clinical Cancer Research, 9, 327-337, 2003, Journal of Medicinal Chemistry., 46: 1116-9,2003; WO 01/060814) (See formula (II) below):

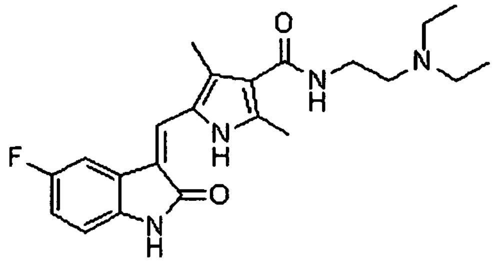

(II)

In the present invention, the angiogenesis inhibitor may form a pharmacologically acceptable salt with acid or base. The above-described angiogenesis inhibitor in the present invention includes such pharmacologically acceptable salts. Examples of salts formed with acid include, but are not limited to, inorganic acid salts such as hydrochlorides, hydrobromates, sulfates and phosphates; and organic acid salts such as formates, acetates, lactates, succinates, fumarates, maleates, citrates, tartrates, stearates, benzoates, methanesulfonates, benzenesulfonates, p-toluenesulfonates and trifluoroacetates. Examples of salts formed with base include, but are not limited to, alkali metal salts such as sodium salts and potassium salts; alkaline earth metal salts such as calcium salts and magnesium salts; organic base salts such as trimethylamine, triethylamine, pyridine, picoline, dicyclohexylamine, N',N'-dibenzylethylenediamine, arginine and lysine; and ammonium salts.

Further, in the present invention, the angiogenesis inhibitor includes the solvates of these compounds and, when these compounds have optical isomers, the solvates thereof and the optical isomers. Examples of the solvate include, but are not limited to, hydrates and non-hydrates. Hydrates are preferable. Examples of solvents include, but are not limited to, water, alcohols (such as methanol, ethanol, n-propanol) and dimethylformamide.

Further, in the present invention, the angiogenesis inhibitor may be in the form of crystal or non-crystal. When there is crystalline polymorphism, the angiogenesis inhibitor may be a single product of any one of the crystal forms or a mixture of such forms.

In the present invention, the angiogenesis inhibitor also includes those angiogenesis inhibitors which undergo metabolism (such as oxidation, reduction, hydrolysis or conjugation) in the body. Further, in the present invention, the

angiogenesis inhibitor also includes those compounds which produce angiogenesis inhibitor in the body as a result of metabolism (such as oxidation, reduction of hydrolysis).

Hereinbelow, the present invention will be described more specifically with reference to the following Examples. However, the present invention is not limited to these Examples.

EXAMPLE 1: Anti-Tumor Effect of Angiogenesis Inhibitor in Human Cancer Cell line Subcutaneously Transplanted Models (*in vivo*)

Human cancer cell lines A375 (purchased from Dainippon Pharma Co., Ltd.), SEKI, HMV-1 (these two lines were purchased from JCRB cell bank, National Institute of Biomedical Innovation), FEM (granted from Dr. Fodstad, The Norwegian Radiumhospital Research Foundation), LOX (purchased from AntiCancer), AZ-521 (purchased from Japan Health Science Foundation), MDA-MB-468, DLD-1, HCT116, SW620, PC-3, DU145, AsPC-1, H526, MDA-MB-231, SK-Mel-2, Lovo and A431 (these 12 lines were purchased from ATCC) were cultured with RPMI1640 (containing 10% FBS) in a 5% $CO_2$ incubator until they reached about 80% confluence. After culturing, cells from each line were recovered with trypsin-EDTA by conventional procedures. The cells were suspended in phosphate buffer solution to prepare a cell suspension of 1 x $10^8$ cells/ml or 5 x $10^7$ cells/ml. Subsequently, 0.1 ml of the cell suspension was subcutaneously transplanted on the lateral side of each nude mouse. After transplantation, when the tumor volume reached about 100-200 $mm^3$, administration of 4-(3-chloro-4-(cyclopropylaminocarbonyl)-aminophenoxy)-7-methoxy-6-quinolinecarboxamide was started (100 mg/kg; twice a day; one week; oral administration). The 4-(3-chloro-4-(cyclopropylaminocarbonyl)-aminophenoxy)-7-methoxy-6-quinolinecarboxamide (a salt of methanesulfonic acid) was prepared based on the disclosure in WO 02/32872 and WO 2005/063713. The major axis and minor axis of tumor were measured with a Degimatic Caliper (Mitsutoyo). Then, tumor volume and ΔT/C were calculated using the following formulas:

$$\text{Tumor volume (TV)} = \text{tumor major axis (mm) x tumor minor axis}^2\ (\text{mm}^2)/2$$

$$\Delta T/C = (\text{tumor volume at day 8 of administration groups - tumor volume at day 1 of administration groups})/(\text{tumor volume at day 8 of control group - tumor volume at day 1 of control group}) \times 100$$

In the above formulas, "day 1" means the day when administration started and "day 8" means the 8th day from the start of the administration.

Based on the intensity of the antitumor effect of 4-(3-chloro-4-(cyclopropylaminocarbonyl)aminophenoxy)-7-methoxy-6-quinoline-carboxamide upon these cancer cell lines, they were classified into shrinkage lines, rest lines and proliferation lines. Cancer cell liness which showed ΔT/C<-30% were classified as shrinkage lines; cancer cell lines which showed -30%<ΔT/C<10% were classified as rest lines; and cancer cell lines which showed 10%<ΔT/C were classified as proliferation lines.

EXAMPLE 2: Preparation and Staining of Tumor Tissue Sections from Human Cancer Cell Line Subcutaneously Transplanted Models; and Correlation between the Antitumor Effect of Angiogenesis Inhibitor and the Ratio of the Number of Blood Vessels Covered with Pericytes to the Total Number of Blood Vessels

Human cancer cell lines A375 (purchased from Dainippon Pharma Co., Ltd.), SEKI, HMV-1 (these two strains were purchased from JCRB cell bank, National Institute of Biomedical Innovation), FEM (granted from Dr. Fodstad, The Norwegian Radiumhospital Research Foundation), LOX (purchased from AntiCancer), AZ-521 (purchased from Japan Health Science Foundation), MDA-MB-468, DLD-1, HCT116, SW620, PC-3, DU145, AsPC-1, H526, MDA-MB-231, SK-Mel-2, Lovo and A431 (these 12 strains were purchased from ATCC) were cultured with RPMI1640 (containing 10% FBS) in a 5% $CO_2$ incubator until they reached about 80% confluence. After culturing, cells from each strain were recovered with trypsin-EDTA by conventional procedures. The cells were suspended in phosphate buffer to prepare a cell suspension of 1 x $10^8$ cells/ml or 5 x $10^7$ cells/ml. Subsequently, 0.1 ml of the cell suspension was subcutaneously transplanted on the lateral side of each nude mouse. After transplantation, when the tumor volume reached about 100-200 $mm^3$, the mice were killed with $CO_2$. Then, the transplanted human tumor was removed by surgical operation. Approximately 5 mm inside of the peripheral part of the tumor tissue was cut with a knife. The thus obtained tumor tissue

was embedded in OCT compound, and then frozen with dry ice to prepare a frozen tissue at -80°C. From the resultant tissue, sections 8 μm in thickness were prepared, mounted on slide glass, washed with running water, and then left stationary in cool acetone at 4° for 10 min. Subsequently, these samples were washed 3 times with 0.1% Tween 20-containing 0.01 M phosphate buffer (hereinafter, called "washing PBS") and reacted in the avidin-blocking solution contained in DAKO Biotin Blockig Kit at room temperature for 10 min. After washing 3 times with the washing PBS, the samples were reacted in the biotin-blocking solution contained in DAKO Biotin Blockig Kit at room temperature for 10 min. After washing in the same manner, the samples were reacted with the normal serum contained in Vector Stain ABC Peroxidase Rat IgG Kit at room temperature for 20 min. After removal of the solution, anti-CD31 antibody (a primary antibody) (designation of the clone: MEC13.3; rat IgG; PharMingen, BD Biosciences) diluted 600-fold with 1% fetal bovine serum-containing 0.1 M phosphate buffer was reacted with the samples at 4°C overnight. After washing, the biotin-labeled secondary antibody contained in Vector Stain ABC Peroxidase Rat IgG Kit was reacted with the samples at room temperature for 30 min. After washing in the same manner, the avidin reagent (mixture of reagents A and B) contained in Vector Stain ABC Peroxidase Rat IgG Kit was further reacted with the samples at room temperature for 30 min. After washing 3 times with 0.01 M phosphate buffer, the samples were subjected to color formation with DAB to thereby stain CD31.

Subsequently, the samples were washed with running water and then washed 3 times with Tris buffer. Alkaline phosphatase-labeled anti-α-SMA antibody (designation of the clone: 1A4; mouse IgG; SIGMA-ALDRICH) diluted 100-fold with Tris buffer was reacted with the samples at room temperature for 1 hr. After washing 3 times with Tris buffer, the fuchsin solution contained in DAKO LSAB Kit (a solution prepared by mixing each two drops of solutions 3 and 4, agitating for 1 min and adding solution 5 to make a 2 ml solution) was added to the samples for color formation to thereby stain α-SMA.

Under microscopic observation, blood vessels in the thus stained samples were counted. Briefly, the number of all the blood vessels and the number of blood vessels covered with pericytes were counted with a CCD camera Hyper Scope (Keyence) at approximately 5 spots per sample. Average values were obtained from the counting results and were converted into the number of blood vessels per unit area and the number of blood vessels covered with pericytes per unit area. Further, the ratio of blood vessels covered with pericytes to the total number of blood vessels was calculated for each of the cancer cell lines and compared to the antitumor effect of 4-(3-chloro-4-(cyclopropylaminocarbonyl)-aminophenoxy)-7-methoxy-6-quinolinecarboxamide. Statistical analysis was performed by Dunnett's multiple comparison test.

From these results, it has become clear that the antitumor effect of 4-(3-chloro-4-(cyclopropylaminocarbonyl) aminophenoxy)-7-methoxy-6-quinolinecarboxamide correlates with the ratio of blood vessels covered with pericytes in tumor tissues (Table 1 and Fig. 1). Therefore, by determining the ratio of blood vessels covered with pericytes in a tumor and using the resultant number as an indicator, it has become possible to predict the antitumor effect of an angiogenesis inhibitor against the relevant tumor without administering the angiogenesis inhibitor to a patient. Thus, the method of the present invention has made it possible to select those patients who are expected to show greater antitumor effect without actually administering an angiogenesis inhibitor to them, enabling to contribute to patients' QOL.

Table 1.

| Cell Line | Type of Cancer | ΔT/C | Classification | Ratio of blood vessels covered with pericytes (%) |
|---|---|---|---|---|
| MDA-MB-468 | Breast cancer | -53% | Shrinkage line | 22.7 |
| MDA-MB-231 | Breast cancer | -33% | Shrinkage line | 6 |
| DU145 | Prostate cancer | -60% | Shrinkage line | 11 |
| AZ-521 | Gastric cancer | -57% | Shrinkage line | 14.6 |
| Lovo | Large bowel cancer | -11% | Rest line | 11 |
| SK-Mel-2 | Melanoma | -2.5% | Rest line | 10 |
| AsPC-1 | Pancreatic cancer | -8% | Rest line | 8 |
| A431 | Epidermoid carcinoma | -8% | Rest line | 10 |
| SW620 | Large bowel cancer | 22% | Proliferation line | 20.6 |
| DLD-1 | Large bowel cancer | 52% | Proliferation line | 29.5 |
| HCT116 | Large bowel cancer | 17% | Proliferation line | 31.6 |
| A375 | Melanoma | 26% | Proliferation line | 20.9 |
| LOX | Melanoma | 42% | Proliferation line | 30 |
| HMVV-1 | Melanoma | 19% | Proliferation line | 24.7 |
| SEKI | Melanoma | 110% | Proliferation line | 18.1 |

(continued)

| Cell Line | Type of Cancer | ΔT/C | Classification | Ratio of blood vessels covered with pericytes (%) |
|---|---|---|---|---|
| FEM | Melanoma | 55% | Proliferation line | 36 |
| PC-3 | Prostate cancer | 54% | Proliferation line | 16 |
| H526 | Small cell lung cancer | 22% | Proliferation line | 81 |

Table 1 shows the antitumor effect of 4-(3-chloro-4-(cyclopropylaminocarbonyl)-aminophenoxy)-7-methoxy-6-quinolinecarboxamide in human cancer cell line transplanted mouse models, the classification of cell lines and the ratio of blood vessels covered with pericytes (%).

EXAMPLE 3: Antitumor Effect of Angiogenesis Inhibitor in Human Cancer Cell Line Subcutaneously Transplanted Models (*in vivo*)

Human cancer cell lines AsPC-1 and H526 (both strains were purchased from ATCC) were cultured with with RPMI1640 (containing 10% FBS) in a 5% $CO_2$ incubator until they reached about 80% confluence. After culturing, cells from each strain were recovered with trypsin-EDTA by conventional procedures. The cells were suspended in phosphate buffer to prepare a cell suspension of 5 x $10^7$ cells/ml. Subsequently, 0.1 ml of the cell suspension was subcutaneously transplanted on the lateral side of each nude mouse. After transplantation, when the tumor volume reached about 50-200 $mm^3$, administration of 5-(5-fluoro-2-oxo-1,2-dihydroindole-3-ylidenemethyl)-2,4-dimethyl-1H-pyrrole-3-carboxylic acid (2-diethylaminoethyl)amide (100 mg/kg, once a day, one week, oral administration) was started. The 5-(5-fluoro-2-oxo-1,2-dihydroindole-3-ylidenemethyl)-2,4-dimethyl-1H-pyrrole-3-carboxylic acid (2-diethylaminoethyl)amide was prepared based on the disclosure in WO 01/060814. The major axis and minor axis of tumor were measured with a Degimatic Caliper (Mitsutoyo). Then, tumor volume and relative tumor volume were calculated using the following formulas:

$$\text{Tumor volume (TV)} = \text{tumor major axis (mm)} \times \text{tumor minor axis}^2\ (\text{mm}^2)/2$$

$$\text{Relative tumor volume (RTV)} = \text{tumor volume on the measurement day/tumor volume on the starting day of administration}$$

The results revealed that, in the 5-(5-fluoro-2-oxo-1,2-dihydroindole-3-ylidenemethyl)-2,4-dimethyl-1H-pyrrole-3-carboxylic acid (2-diethylaminoethyl)amide (Compound 2) administration group, tumor proliferation rested in AsPC-1 which was classified as a rest cell line in Example 1 and was delayed in H526 which was classified as a proliferation cell line in Example 1, as observed similarly in the 4-(3-chloro-4-(cyclopropylaminocarbonyl)aminophenoxy)-7-methoxy-6-quinoline-carboxamide (Compound 1) administration group (Fig. 2). This means that Compound 2 produces antitumor effect to an extent similar to that extent of the antitumor effect produced by Compound 1. Therefore, it has become clear that not only the antitumor effect of Compound 1 but also the antitumor effect of other angiogenesis inhibitors can be predicted by using as an indicator the ratio of blood vessels covered with pericytes (Fig. 2). This means that it has become possible to predict the antitumor effect of an angiogenesis inhibitor by determining the number of those blood vessels which are covered with pericytes in a tumor and using the resultant number as an indicator, without administering the angiogenesis inhibitor to patient. For this reason, the method of the present invention is capable of selecting those patients who are expected to show greater antitumor effect without administering the angiogenesis inhibitor to these patients and thus contributes to patients' QOL.

EXAMPLE 4: Correlation between the Antitumor Effect of Angiogenesis Inhibitor and the Ratio of the Number of Blood Vessels Covered with Pericytes in Human Cancer Cell Line Subcutaneously Transplanted Models (Quantitative RT-PCR)

1. Purification of Total RNA from Tumor Tissue in Human Cancer Cell Line Subcutaneously Transplanted Models

Human cancer cell lines MDA-MB-468, DLD-1, HCT116, SW620, PC-3, DU145, AsPC-1, H526, MDA-MB-231, MDA-MB-435, SK-OV-3, Lovo, 7860, 22Rv (these 14 strains were purchased from ATCC), HMV-1 (purchased from JCRB cell bank, National Institute of Biomedical Innovation), Colo320DM, A549, A375 (these 3 strains were purchased from Dainippon Pharma Co., Ltd.), FEM (granted from Dr. Fodstad, The Norwegian Radiumhospital Research Foundation) and LOX (purchased from AntiCancer) were cultured with RPMI1640 (containing 10% FBS) in a 5% $CO_2$ incubator until they reached about 80% confluence. After culturing, cells from each line were recovered with trypsin-EDTA by conventional procedures. The cells were suspended in phosphate buffer to prepare a cell suspension of 1 x $10^8$ cells/ml

or 5 x $10^7$ cells/ml. Subsequently, 0.1 ml of the cell suspension was subcutaneously transplanted on the lateral side of each nude mouse. After transplantation, when the tumor volume reached about 100-200 $mm^3$, mice were killed with $CO_2$ and the transplanted human tumor was removed from each mouse by surgical operation. The removed tumor tissue was divided into two portions and individual weights were measured. For one of these two portions, 1 ml of TRIZOL reagent (Invitrogen) was added per 50 mg of the tumor. Then, the tumor was homogenized and stored at -20°C.

Subsequently, 0.2 ml of chloroform (purchased from Junsei Chemical) was added per 1 ml of TRIZOL reagent. The resultant solution was shook and agitated vigorously for 15 sec, left at room temperature for 2-3 min, and then centrifuged (12,000xg, 10 min, 4°C). After centrifugation, the aqueous layer was tranferrred to a fresh tube. To this tube, 0.2 ml of isopropyl alcohol (Wako Pure Chemical Industries) was added per 1 ml of the RIZOL reagent used. After leaving at room temperature for 10 min, the tube was centrifuged (12,000xg, 10 min, 4°C). The resultant precipitate was washed with 75% ethanol (Wako Pure Chemical Industries) and air dried. The thus obtained total RNA was subjected to the subsequent determination.

2. Quantitative Determination of RNA

Quantitative RT-PCR was performed as described below using gene-specific probes (TaqMan Gene Expression Assays Mixture (ASSAYS-ON-DEMAND); Applied Biosystems) and ABI Prism 7900 Sequence Detection System (Perkin-Elmer Applied Biosystems).

Operation was performed in two-stages, i.e., reverse transcription reaction and PCR reaction. Reverse transcription reaction (the first stage) was performed by adding to 3 μl of the resultant RNA (100 ng/μl), 6 μl of dNTP, 1.5 μl of oligo $d(T)_{16}$ primer, 0.6 μl of RNase Inhibitor, 0.75 μl of Multiscribe Reverse Transcriptase, 6.6 μl of 25 mM $MgCl_2$ (Perkin-Elmer Applied Biosystems) and 6 μl of DEPC water, retaining the mixture at 25°C for 10 min and then heating at 48°C for 30 min. The reaction was terminated by heating the reaction solution at 95°C for 5 min to thereby obtain a cDNA solution for PCR.

The thus obtained cDNA was subjected to the PCR reaction at the second stage. The PCR reaction was performed in a reaction system comprising 5 μl of cDNA solution for PCR diluted 5-fold with DEPC water, 6.25 μl of TaqMan Universal PCR Master Mix, 0.625 μl of 200 nM TaqMan Gene Expression Assays probe and 0.625 μl of $H_2O$. The reaction conditions were as follows: 50°C for 2 min and 95 °C for 10 min, followed by 40 cycles of 95°C for 20 sec, 55°C for 20 sec and 72°C for 30 sec. With respect to probes and primers, TaqMan Gene Expression Assays mixture (ASSAYS-ON-DEMAND; Mm00802455_s1; Applied Biosystems) was used for determining desmin, and TaqMan Gene Expression Assays mixture (ASSAYS-ON-DEMAND; Mm00607939_sl; Applied Biosystems) was used for determining β-actin.

3. Methods of Data Analysis

For quantitative analysis of individual genes, calibration curves were prepared using mRNA samples of SK-OV-3. Gene expression levels in individual cancer cell lines were determined by calculating Ct (abbreviation of threshold cycle value which means a number of PCR cycles required for a PCR product to reach a specific concentration) from the calibration curve. The expression level of desmin in each cancer cell line amended by the β-actin expression level was taken as the expression level ratio of desmin in each cancer cell line, which was used in comparative analysis. With respect to the classification of individual cell lines, the classification made in Example 1 was used. It should be noted that shrinkage cell lines and rest cell lines were put into one group together and classified as sensitive cell lines.

Comparison between sensitive cell lines and proliferation cell lines was performed by permutation test. When P was $<0.05$, it was judged as significantly different.

The results revealed that expression of desmin was significantly higher in proliferation cell lines (5.6) than in sensitivity cell lines (2.5) (Fig. 3).

From these results, it has become possible to predict the antitumor effect of an angiogenesis inhibitor by determining the ratio of those blood vessels covered with pericytes in a tumor using as an indicator the expression of a pericyte marker (such as desmin), and then using the number of those blood vessels as an indicator.

INDUSTRIAL APPLICABILITY

According to the present invention, a method of predicting the antitumor effect of an angiogenesis inhibitor has been provided.

More specifically, it has become possible to predict the antitumor effect of an angiogenesis inhibitor by determining the number of those blood vessels which are covered with pericytes in a tumor and using the determined number as an indicator.

Since the method according to the present invention has made it possible to predict the antitumor effect of an

angiogenesis inhibitor without administering the angiogenesis inhibitor to patients, the method allows selection of those patients who are expected to show greater antitumor effect and enables contribution to patients' QOL.

## Claims

1. A method of predicting the antitumor effect of an angiogenesis inhibitor, comprising the following steps:

   a first step of determining the number of blood vessels in a tumor sample that has been removed from a cancer patient and the number of those blood vessels which are covered with pericytes in the tumor sample; and
   a second step of judging that a cancer patient is highly sensitive to the angiogenesis inhibitor when the ratio of the number of those blood vessels covered with pericytes in the tumor to the total number of blood vessels in the tumor is 25% or less,
   wherein the angiogenesis inhibitor is one compound selected from the group consisting of:

   4-(3-chloro-4-(cyclopropylaminocarbonyl)aminophenoxy)-7-methoxy-6-quinolinecarboxamide;
   4-(3-chloro-4-(ethylaminocarbonyl)aminophenoxy)-7-methoxy-6-quinolinecarboxamide;
   N6-methoxy-4-(3-chloro-4-(((cyclopropylamino)carbonyl)amino)phenoxy)-7-methoxy-6-quinolinecarboxamide;
   4-(3-chloro-4-(methylaminocarbonyl)aminophenoxy)-7-methoxy-6-quinolinecarboxamide;
   N6-methoxy-4-(3-chloro-4-(((ethylamino)carbonyl)amino)phenoxy)-7-methoxy-6-quinolinecarboxamide;
   5-(5-fluoro-2-oxo-1,2-dihydroindole-3-ylidenemethyl)-2,4-dimethyl-1H-pyrrole-3-carboxylic acid (2-diethylaminoethyl)amide;

   or a pharmacologically acceptable salt of said compound, or a solvate of said compound or said salt.

2. The method according to claim 1, wherein, in the second step, the patient is judged to be highly sensitive to the compound when the ratio of the number of those blood vessels covered with pericytes in the tumor to the total number of blood vessels in the tumor is 20% or less.

3. The method according to claim 1, wherein, in the second step, the patient is judged to be highly sensitive to the compound when the ratio of the number of those blood vessels covered with pericytes in the tumor to the total number of blood vessels in the tumor is 15% or less.

## Patentansprüche

1. Verfahren zum Vorhersagen der Antitumorwirkung eines Angiogenesehemmers, umfassend folgende Schritte:

   einen ersten Schritt des Bestimmens der Anzahl von Blutgefäßen in einer Tumorprobe, die einem Krebspatienten entnommen wurde, und die Anzahl der Blutgefäße in der Tumorprobe, die mit Perizyten bedeckt sind; und
   einen zweiten Schritt des Beurteilens, dass ein Krebspatient hochempfindlich gegen den Angiogenesehemmer ist, wenn das Verhältnis der Anzahl der Blutgefäße in dem Tumor, die mit Perizyten bedeckt sind, zu der Gesamtzahl der Blutgefäße in dem Tumor 25 % oder weniger beträgt,
   wobei der Angiogenesehemmer eine Verbindung ist, ausgewählt aus der Gruppe bestehend aus:

   4- (3-Chlor-4-(cyclopropylaminocarbonyl)aminophenoxy)-7-methoxy-6-chinolincarboxamid;
   4-(3-Chlor-4-(ethylaminocarbonyl)aminophenoxy)-7-methoxy-6-chinolincarboxamid;
   N6-Methoxy-4-(3-chlor-4-(((cyclopropylamino)carbonyl)amino)phenoxy)-7-methoxy-6-chinolincarboxamid;
   4-(3-Chlor-4-(methylaminocarbonyl)aminophenoxy)-7-methoxy-6-chinolincarboxamid;
   N6-Methoxy-4-(3-chlor-4-(((ethylamino)carbonyl)amino)phenoxy)-7-methoxy-6-chinolincarboxamid;
   5-(5-Fluor-2-oxo-1,2-dihydroindol-3-ylidenmethyl)-2,4-dimethyl-1H-pyrrol-3-carbonsäure(2-diethylaminoethyl)amid;

   oder ein pharmakologisch verträgliches Salz der Verbindung oder ein Solvat der Verbindung oder des Salzes.

2. Verfahren gemäß Anspruch 1, wobei bei dem zweiten Schritt der Patient als hochempfindlich gegen die Verbindung

beurteilt wird, wenn das Verhältnis der Anzahl der Blutgefäße in dem Tumor, die mit Perizyten bedeckt sind, zu der Gesamtzahl der Blutgefäße in dem Tumor 20 % oder weniger beträgt.

**3.** Verfahren gemäß Anspruch 1, wobei bei dem zweiten Schritt der Patient als hochempfindlich gegen die Verbindung beurteilt wird, wenn das Verhältnis der Anzahl der Blutgefäße in dem Tumor, die mit Perizyten bedeckt sind, zu der Gesamtzahl der Blutgefäße in dem Tumor 15 % oder weniger beträgt.

## Revendications

**1.** Procédé de prédiction de l'effet antitumoral d'un inhibiteur d'angiogénèse, comprenant les étapes suivantes :

une première étape consistant à déterminer le nombre de vaisseaux sanguins dans un prélèvement de tumeur qui a été prélevé chez un patient atteint de cancer et le nombre de ces vaisseaux sanguins qui sont recouverts de péricytes dans le prélèvement de tumeur ; et
une deuxième étape qui consiste à juger si un patient atteint de cancer est très sensible a l'inhibiteur d'angiogénèse lorsque le rapport entre le nombre de ces vaisseaux recouverts de péricytes dans la tumeur et le nombre total de vaisseaux sanguins dans la tumeur est de 25 % ou inférieur,
l'inhibiteur d'angiogénèse étant un composé choisi dans le groupe constitué de :

4- (3-chloro-4-(cyclopropylaminocarbonyl)aminophénoxy)-7-méthoxy-6-quinoléinecarboxamide ;
4- (3-chloro-4-(éthylaminocarbonyl)aminophénoxy)-7-méthoxy-6-quinoléinecarboxamide ;
N6-méthoxy-4-(3-chloro-4-(((cyclopropylamino)carbonyl)amino)phénoxy)-7-méthoxy-6-quinoléinecarboxamide ;
4- (3-chloro-4-(méthylaminocarbonyl)aminophénoxy)-7-méthoxy-6-quinoléinecarboxamide ;
N6-méthoxy-4-(3-chloro-4-(((éthylamino)carbonyl)amino)phénoxy)-7-méthoxy-6-quinoléinecarboxamide ;
(2-diéthylaminoéthyl)amide d'acide 5-(5-fluoro-2-oxo-1,2-dihydroindol-3-ylidèneméthyl)-2,4-diméthyl-1H-pyrrole-3-carboxylique ;

ou un sel pharmacologiquement acceptable dudit composé, ou un solvate dudit composé ou dudit sel.

**2.** Procédé selon la revendication 1, **caractérisé en ce que**, dans la deuxième étape, le patient est jugé très sensible au composé lorsque le rapport entre le nombre des vaisseaux sanguins recouverts de péricytes dans la tumeur et le nombre total de vaisseaux sanguins dans la tumeur est de 20 % ou inférieur.

**3.** Procédé selon la revendication 1, **caractérisé en ce que**, dans la deuxième étape, le patient est jugé très sensible au composé lorsque le rapport entre le nombre des vaisseaux sanguins recouverts de péricytes dans la tumeur et le nombre total de vaisseaux sanguins dans la tumeur est de 15 % ou inférieur.

Fig. 1

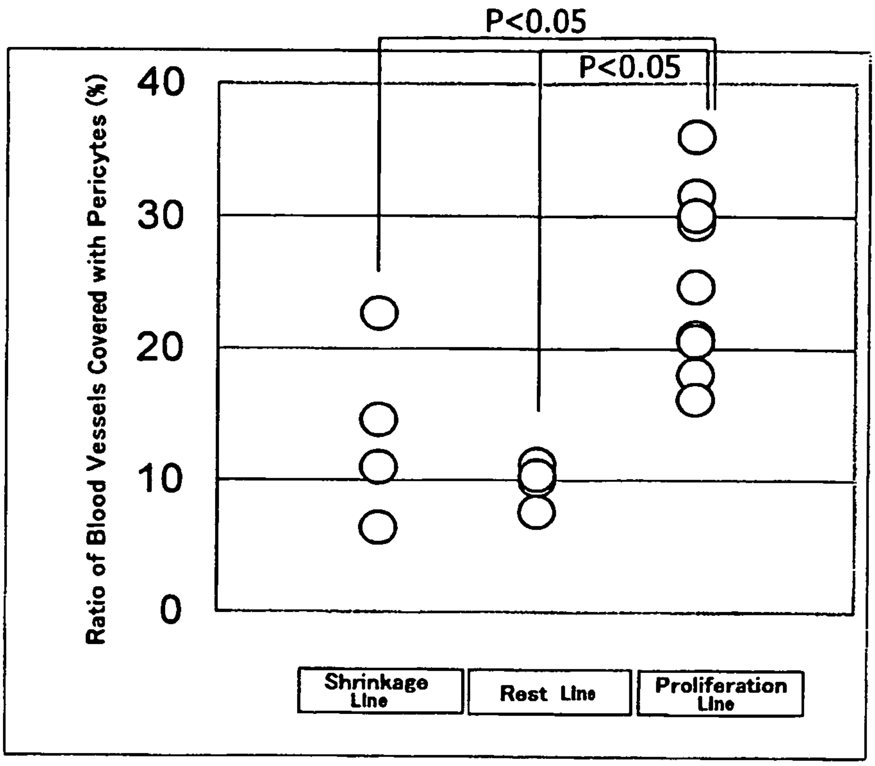
P<0.05
P<0.05
Ratio of Blood Vessels Covered with Pericytes (%)
40
30
20
10
0
Shrinkage Line
Rest Line
Proliferation Line

Fig. 2

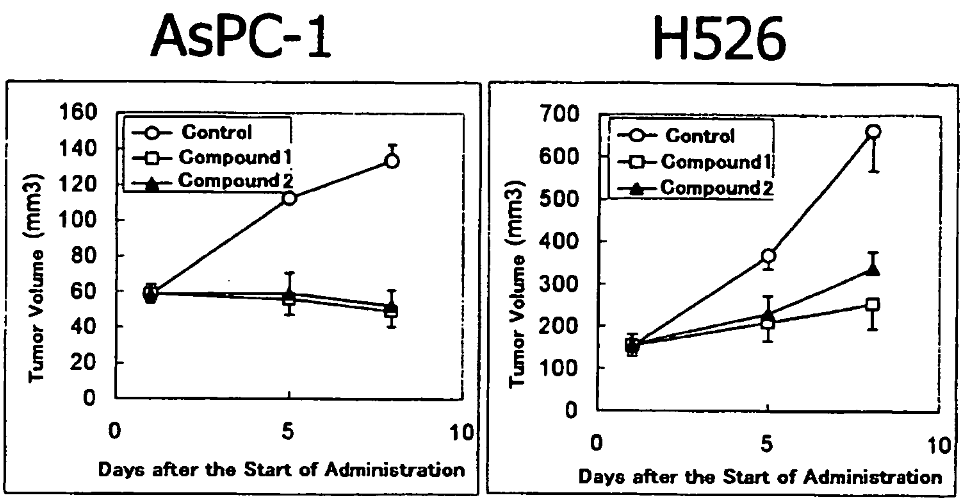
AsPC-1
H526
Control
Compound 1
Compound 2
Tumor Volume (mm3)
Days after the Start of Administration
160
140
120
100
80
60
40
20
0
700
600
500
400
300
200
100
0
5
10

Fig. 3

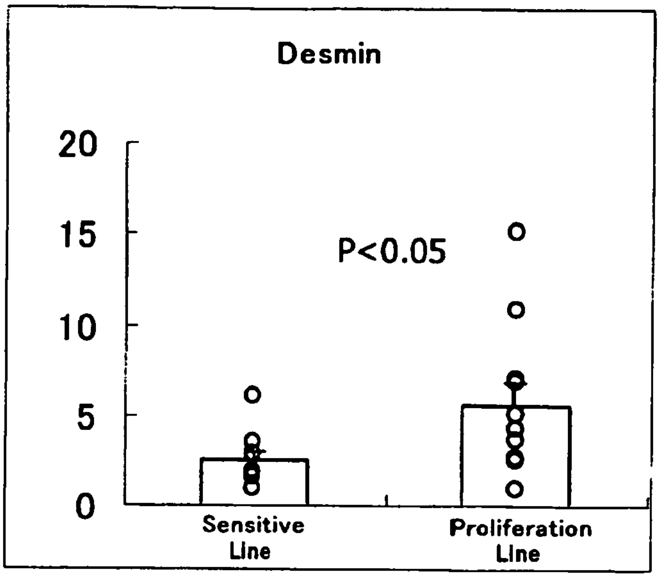

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0232872 A
- WO 2004080462 A
- WO 2005063713 A
- WO 01060814 A


### Non-patent literature cited in the description


- **BENAJMIN et al.** *J. Clin. Invest.,* 1999, vol. 103, 159-165
- Bevacizumab plus irinotecan, fluorouracil, and leucovorin for metastatic colorectal cancer. *N Engl J Med.,* 2004, vol. 350, 2335-42
- Inhibition of vascular endothelial growth factor (VEGF) signaling in cancer causes loss of endothelial fenestrations, regression of tumor vessels, and appearance of basement membrane ghosts. *Am J Pathol.,* 2004, vol. 165, 35-52
- Direct evidence that the VEGF-specific antibody bevacizumab has antivascular effects in human rectal cancer. *Nature Medicine,* 2004, vol. 10, 145-147
- Dynamic contrast-enhanced magnetic resonance imaging as a biomarker for the pharmacological response of PTK787/ZK 222584. *J Clin Oncol.,* 2003, vol. 21, 3955-64
- Characterization of smooth muscle cell and pericyte differentiation in the rat retina in vivo, Investigative. *Ophthalmology. Visual Science,* 2004, vol. 45, 2795-2806
- Cellular abnormalities of blood vessels as targets in cancer. *Current Opinion in Genetics and Development,* 2005, vol. 15, 102-111
- A new method for isolation of smooth muscle cells from human umbilical cord arteries. *Scand J. Clin. Lab. Invest.,* 1997, vol. 57, 21-29
- Localization of Brain Endothelial Luminal and Ablwninal Transporters with Immunogold Electron Microscopy. *NeuroRx.,* 2005, vol. 2, 27-43
- Jikkenn Igaku Bessatu, Shin-Idenishikogaku Handbook. Yodosha, 2003
- Saibo-Kogaku Bessatu, Me de Mirujikkenn note series, Bio-Jikkenn Illustrated Vol. 5, Tanpaku-nante-Kowakunai. *Immunostaining,* 1997, vol. 5, 127-163
- Vascular and haematopoietic stem cells: novel targets for anti-angiogenesis therapy?. *Nature Reviews Cancer,* 2002, vol. 2, 826-35
- *Clinical Cancer Research,* 2003, vol. 9, 327-337
- *Journal of Medicinal Chemistry,* 2003, vol. 46, 1116-9